(19) Europäisches Patentamt
European Patent Office
Office européen des brevets

(11) EP 1 923 077 B1

(12) EUROPÄISCHE PATENTSCHRIFT

(45) Veröffentlichungstag und Bekanntmachung des Hinweises auf die Patenterteilung:
**12.01.2011 Patentblatt 2011/02**

(51) Int Cl.:
*A61L 15/26* (2006.01)     *A61L 15/60* (2006.01)

(21) Anmeldenummer: **06023099.2**

(22) Anmeldetag: **07.11.2006**

(54) **Mehrschichtige, absorbierende Wundauflage mit einer hydrophilen Wundkontaktschicht**

Multilayer, absorbent wound dressing with a hydophilic wound contact layer

Pansement multicouche absorbant avec une couche hydrophile en contact de plaies

(84) Benannte Vertragsstaaten:
**AT BE BG CH CY CZ DE DK EE ES FI FR GB GR HU IE IS IT LI LT LU LV MC NL PL PT RO SE SI SK TR**

(43) Veröffentlichungstag der Anmeldung:
**21.05.2008 Patentblatt 2008/21**

(73) Patentinhaber: **Paul Hartmann AG**
**89522 Heidenheim (DE)**

(72) Erfinder: **Eckstein, Axel, Dr.**
**89522 Heidenheim (DE)**

(56) Entgegenhaltungen:
**EP-A1- 0 059 049     EP-A1- 0 097 517**
**EP-A1- 0 691 113     WO-A-02/38097**
**WO-A-02/45761     WO-A-89/01346**
**GB-A- 2 228 682     JP-A- 6 078 948**

## Beschreibung

Die vorliegende Erfindung betrifft Wundauflagen, insbesondere zur Behandlung von mittel bis stark exsudierenden Wunden, sowie deren Verwendung in der modernen Wundbehandlung.

Die Heilung von Hautwunden beruht auf der Fähigkeit der Haut zur Regeneration von Epithelen sowie von Binde und Stützgewebe. Sie ist als ein komplexes Geschehen ineinander übergreifender Zellaktivitäten gekennzeichnet, die den Heilungsprozess schrittweise vorantreiben. So werden in der Literatur unabhängig von der Art der Wunde drei wesentliche Heilungsphasen einer Wunde beschrieben. Hierzu gehört die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differentierungsphase zur Epithelisierung und Narbenbildung (Phase 3, Epithelisierungsphase).

Zur Unterstützung der einzelnen Wundheilungsphasen sind zahlreiche Vorschläge in der Literatur beschrieben. So beschreiben die europäischen Patente EP 59048, EP 59049, EP 97517, EP 99748 und EP 106439 jeweils eine nicht wundverklebende Wundauflage, die als absorbierende Lage einen Polyurethan-Schaum aufweist. Als Wundkontaktschicht umfassen diese Wundauflagen ein elastomeres Netz oder einen gelochten Film aus einem Polyurethan.

Mit der EP 486522 wird eine flexible Wundauflage beansprucht, die eine im anwendungsgerechten Zustand der Wunde gegenüberliegende, ebene Lage eines Schaummaterials, vorzugsweise einen Polyurethan-Schaum umfasst, die wiederum mit einer diskontinuierlichen Klebeschicht versehen ist. Auf der wundabgewandten Seite weist die Wundauflage ein wasserundurchlässiges aber wasserdampfdurchlässiges Material vorzugsweise einen Polyurethanfilm auf. Dabei sollen mindestens zwei gegenüberliegende Seiten der Wundauflage abgeflachte Ränder aufweisen

Mit de WO 94/07935 wird eine Wundauflage beschrieben, die aus einem selbstklebenden hydrophilen Polyurethangelschaum besteht, der erhältlich ist aus einer Polyhydroxyverbindung, einem Polyisocyanat, einem nichtwässrigem Schäumungsmittel und einem Wasser absorbierenden Superabsorber.

Darüber hinaus wird mit WO 97/42985 eine Wundauflage beschrieben; die eine absorbierende Schaumschicht und ein darauf aufgebrachtes klebendes, hydrophobes Gel umfasst. Das Schaummaterial ist porös oder gelocht, wobei die Löcher im anwendungsgerechten Zustand der Wundauflage proximal zur Wunde liegen und die Wände der Löcher mit dem Gel beschichtet sind.

Weiterhin wird mit der WO 2004/060359 eine Wundauflage beschrieben, die ein mit Ausnehmungen versehenes absorbierendes Schaummaterial umfasst. In den Ausnehmungen sind Superabsorberpartikel eingebracht, wobei die Ausnehmungen mittels einer Rückenschicht verschlossen sind. Auf der im anwendungsgerechten Zustand zur Wunde weisenden Oberfläche des absorbierenden Schaummaterials ist ein hydophobes, elastomeres Silikongel aufgebracht.

Insgesamt sind im Stand der Technik Wundauflagen beschrieben, die als Wundkontaktschicht ein hydrophobes Gel oder einen hydrophilen Schaum umfassen.

Ausgehend von dem Stand der Technik ist es daher eine Aufgabe der vorliegenden Erfindung eine alternative und verbesserte Wundauflage zur Behandlung von sezernierenden Wunden bereitzustellen, die insbesondere in der Reinigungsphase oder der Granulationsphase der Wundheilung eingesetzt werden kann. Diese Wundauflage soll nicht wundverklebende Eigenschaften besitzen, den Granulationsprozess einer Wunde unterstützen sowie gleichzeitig die Mazeration der die Wunde umgebenden Haut verhindern.

Überraschenderweise wird diese Aufgabe durch eine mehrschichtige Wundauflage gemäß Anspruch 1 gelöst, die eine Trägerschicht, ein absorbierende Schicht und eine hydrophile Wundkontaktschicht umfasst, wobei die Wundkontaktschicht mit der absorbierenden Schicht verbunden ist und ein hydrophiles Polyurethan-Elastomer umfasst. Insbesondere besteht die Wundkontaktschicht aus einem hydrophilen Polyurethan-Elastomer.

Ein besonderer Vorteil dieser Wundauflage besteht darin, dass durch die Wundkontaktschicht einerseits eine Abstandsschicht zwischen der absorbierenden Schicht und einer Wunde geschaffen Wird und somit auch ansonsten zur Wundverklebung neigende Materialien als absorbierende Schicht verwendet werden können, und andererseits durch das hydrophile Polyurethan-Elastomer eine nicht wundverklebende Wundkontaktschicht geschaffen wird, die durch ihre Hydrophilie einen verbesserten Transport von Wundflüssigkeit von der Wunde zur absorbierenden Schicht erlaubt. Darüber hinaus hat sich gezeigt, dass diese Wundauflage die Mazeration, das heißt, die Aufweichung und damit verbundene Schädigung der die Wunde umgebenden Haut verhindert oder zumindest einschränkt wird. Somit kann eine Wundauflage bereitgestellt werden, die die Wunde umgebende Haut schont und die im hohen Maße die Wundheilung fördert.

Hierbei soll im Zusammenhang mit der vorliegenden Erfindung unter einem Polyurethan-Elastomer eine elastomere Verbindung verstanden sein, die aus mindestens einem Di- oder Polyisocyanat (Isocyanat-Komponente) und mindestens einem Diol- oder Polyol (Polyol-Komponente) gemäß Anspruch 1 durch Polyadditionsreaktionen herstellbar ist, ohne dass die hydrophile Kontaktschicht oder das Elastomer als Schaum vorliegt. Als geeignete Isocyantkomponente oder Polyolkomponente können sowohl präpolymere Verbindungen als auch monomere Verbindungen eingesetzt werden.

Beispiele geeigneter, erfindungsgemäßer Di- oder Polyisocyanate sind Dicyclohexylmethandiisocyanat, Butan-1,4-diisocyanat, Tetramethoxybutan-1,4-diisocyanat, Hexan-1,6-diisocyanat, Ethylendiisocyanat, 2,2,4-Trimethylhexamathylendiisocyanat, Ethylethylendiisocyanat, Dicyclohexylmethandiisocyanat.1,4-Diisocyanatocyclohexan,1,3-Diisocyanatocyclohexan, 1,2-Diisocyanatocyclohexan, 1,3-Diisocyanatocyclopentan, 1,2-Diisocyanatocyclopentan, 1,2-Diisocyanatocyclobutan,1-Isocyanatomethyl-3-isocyanato-1,5,5-trimethylcyclohexan (Isophoron-diisocyanat, IPDI), 1-Methyl-2,4-diisocyanatocyclohexan, 1,6-Diisocyanato-2,2,4-trimethylhexan, 1,6-Diisocyanato-2,4,4-trimethylhexan, 5-Isocyanato-1-(2-isocyanatoeth-1-yl)-1,3,3-trimethylcyclohexan, 5-Isocyanato-1-(3-isocyanatoprop-1-yl)-1,3,3-trimethylcyclohexan, 5-Isocyanato-1-(4-isocyanatobut-1-yl)-1,3,3-trimethylcyclohexan, 1-Isocyanato-2-(3-isocyanatoprop-1-yl)-cyclohexan, 1-Isocyanato-2-(2-isocyanatoeth-1-yl)-cyclohexan, 2-Heptyl-3,4-bis(9-isocyanatononyl)-1-pentylcyclohexan, Norbonandiisocyanatomethyl, chlorierte, bromierte, schwefel- oder phosphorhaltige aliphatische oder cycloaliphatische Diisocyanate, sowie Derivate der aufgeführten Diisocyanate, insbesondere dimerisierte oder trimerisierte Typen.

Gemäß der Erfindung werden hierbei Polyurethan-Elastomere aus aliphatischen oder cycloaliphatische Di- oder Polyisocyanaten hergestellt. Insbesondere sind dabei linear aliphatische oder cycloaliphatische Diisocyanate bevorzugt, wobei weiterhin fünf- oder sechsgliedrige cycloaliphatische Diisocyanate bevorzugt sind. In einer besonders bevorzugten Ausführungsform wird Isophorondiisocyanat als sechsgliedriges cycloaliphatisches Diisocyanat verwendet, welches sehr gute Wasserdampfdurchlässigkeiten zulässt

Weiterhin bevorzugt sind als Isocyanat-Komponente Präpolymere aus aliphatischen oder cycloaliphatischen Di- oder Polyisocyanaten und Di- oder Polyolen, weiterhin bevorzugt Präpolymere aus cycloaliphatischen Diisocyanaten, wobei insbesondere als Polyole Polyetherpolyole oder Polyesterpolyole Verwendung finden.

In der Regel weisen die im Rahmen der vorliegenden Erfindung einsetzbaren Polyurethan-Präpolymeren ein Molekulargewicht von etwa 500 g / mol bis etwa 15000 g / mol, vorzugsweise etwa 500 g / mol bis etwa 10000 g / mol, besonderes bevorzugt etwa 700 g / mol bis etwa 4500 g / mol auf.

Beispiele geeigneter erfindungsgemäßer Diole oder Polyole sind Oxyalkyl-Polymere, vorzugsweise 2, 3, 4, 5 oder 6 Hydroxylgruppen aufweisende Polyetherpolyole mit OH-Zahlen von 20 bis 112 und einem Ethylenoxid-Gehalt von ≥ 10 Gew.%, vorzugsweise 10 bis 40 Gew.%, besonders bevorzugt 10 bis 20 Gew.%. Glasübergangstemperaturen sollten hierbei möglichst tief liegen, also unter etwa 20 °C, vorzugsweise unter etwa 0 °C, besonders bevorzugt unter etwa -10 °C.

Polyetherpolyole mit Molekulargewichten zwischen 600 und 12000 sind bevorzugt und können nach bekannten Verfahren bspw. durch Umsetzung der Starterverbindungen mit einem reaktiven H-Atom mit Alkylenoxiden (beispielsweise Ethylen-, und/oder Propylenoxid, vorzugsweise Propylenoxid, Butylenoxid, Styroloxid, Tetrahydrofuran oder Epichlorhydrin oder Gemischen aus zwei oder mehr davon) erhalten werden. Ebenfalls sind Tetramethylenetherglykole einsetzbar. Ebenfalls möglich sind weitere Modifikationen z.B. mit Monoethylenglycol (MEG), Dipropylenglykol (DPG), Trimethylolpropan (TMP). Bevorzugt für den Einsatz in der Medizin werden heute aliphatische Polyetherpolyole eingesetzt.

Geeignete Startverbinduhgen sind beispielsweise Wasser, Ethylenglykol, Propylenglykol-1,2 oder-1,3, Butylenglykol-1,4 oder-1,3, Hexandiol-1,6, Octandiol-1,8 Pentandiol-1,5, Heptandiol-1,7, und deren höhere Homologe, Neopentylglykol, 1,4-Hydroxymethylcyclohexan, 2-Methyl-1,3-propandiol, Glyzerin, Trimethylolpropan, 2,2-(bis-4,4'-hydroxyphenyl)-Propan, Trimethylolpropan, Glyzerin oder Pentaerythrit, Hexantriol-1,2,6, Butantriol-1,2,4 Trimethylolethan, Mannitol, Sorbitol, Methylglykoside, Zucker, Phenol, Isononylphenol, Resorcin, Hydrochinon, 1,2,2- oder 1,1,2-Tris-(hydroxyphenyl)-ethan, Ammoniak, Methylamin, Ethylendiamin, Tetra- oder Hexamethylenamin, Triethanolamin, Anilin, Phenylendiamin, 2,4- und 2,6-Diaminotoluol und Polyphenylpolymethylenpolyamine, wie sie sich durch Anilin-Formaldehydkondensation erhalten lassen, oder Gemische aus den vorstehenden Startverbindungen davon.

Auch können die vorgenannten Diole oder Polyole gemischt werden. Dabei ist die Verträglichkeit zu berücksichtigen. Bevorzugt werden im Zusammenhang mit der vorliegenden Erfindung aliphatische Polyesterpolyole eingesetzt.

Gegebenenfalls kann das erfindungsgemässe Polyurethan-Elastomer noch Zusatzstoffe enthalten wie beispielsweise Weichmacher, Stabilisatoren wie Antioxidantien oder Photostabilisatoren, Tackifier, Farbstoffe, Füllstoffe, Verdicker, Rheologieaddtive.

Als Weichmacher werden beispielsweise Phthalsäurederivate eingesetzt, beispielsweise Phthatsäureester, welche 6 bis 12 Kohlenstoffatome aufweisen und mit einem linearen Alkanol verestert wurden, z.B. Dioctylphthalat. Polyethylengykole und deren Derivate, pflanzliche und tierische Öle, wie Glycerinester von Fettsäuren und deren Polymerisationsprodukte und Benzoatverbindungen (Benzoatweichmacher, beispielsweise Sucrosebenzoat, Diethylenglykoldibenzoat und/oder Diethylenglykolbenzoat, bei dem etwa 50 bis etwa 95% aller Hydroxylgruppen verestert worden sind, Phosphat-Weichmacher beispielsweise t-Butylphenyldiphenylphosphat, Polyethylenglykole und deren Derivate, beispielsweise Diphenylether von Poly(ethylenglykol), flüssige Harzderivate, beispielsweise der Methylester von hydriertem Harz sind ebenfalls als Weichmacher geeignet. Besonders bevorzugt sind aliphatische Diester wie Adipin- oder Sebacindinonylester.

Zu den im Rahmen der Erfindung eingesetzten Stabilisatoren (Antioxidantien) zählen gehinderte Phenole wie BHT, Irganox® 1010, 1076, 1330, 1520 (Ciba Speciality Chemicals) sowie Tocopherole. Besonders bevorzugt eingesetzt wird Vitamin E (alpha-Tocopherol). Ebenfalls können polyfunktionelle Phenole sowie schwefel- und phoshorhaltige Verbindungen und/oder 1,3,5-Trimethyl-2,4,6-tris(3,5-di-tert-butyl-4-hydro-xybenzyl)benzol; Pentaerythrittetrakis-3-(3,5-di-tert-butyl-4-hydroxyphenyl)propionat; n-Octadecyl-3,5-di-tert-butyl-4-hydroxyphenyl)propionat; 4,4-Methylen-bis(2,6-di-tert-butyl-phenol); 4,4-Thiobis(6-tert-butyl-o-cresol); 2,6-Di-tert-butylphenol; 6-(4-Hydroxyphonoxy)-2,4-bis(n-octylthio)-1,3,5-triazin; Di-n-Octadecyl-3,5-di-tert-butyl-4-hydroxybenzyl-phos-phonate; 2-(n-Octylthio)ethyl-3,5-di-tert-butyl-4-hydroxy-ben-zoat; und Sorbithexa[3-(3,5-di-tertbutyl-4-hydroxy-phenyl)-propionat] zum Einsatz kommen. Als Photostabilisatoren sind zum Beispiel Tinuvin®-Produkte (Ciba Speciality Chemicals), Benzotriazol-Verbindungen, Salicylate, substituierte Tolyl- und Metall-Chelat-Verbindungen geeignet, wobei Benzotriazol-Derivate bevorzugt werden. Kombinationen der oben genannten Verbindungen sind auch möglich. Die üblicherweise eingesetzten Mengen sind zwischen 0,1 und 10 Gew.%.

Für die Einstellung bestimmter Eigenschaften des Polyurethan-Elastomers können fachüblich weitere Zusatzstoffe verwendet werden. Darunter fallen beispielsweise Farbstoffe wie Titandioxid, Füllstoffe wie Talkum, Kreide, Ton und dergleichen. Es ist ebenfalls möglich, bestimmte hydrophile Polymere einzubauen, beispielsweise, PVOH (Polyvinylalkohol), Polyvinylpyrrolidon, Hydroxypropylcellulose, Polyvinylmethylether und Celluloseester, speziell deren Acetate mit geringem Substitutionsgrad. Diese können die Benetzbarkeit der Polyurethan-Elastomers erhöhen. Unter Füllstoffen werden die in der Polyurethanchemie üblicherweise eingesetzten Füllstoffe verstanden. Darunter zählen auch Zinkoxid, Titanoxid und Kieselsäurederivate (z.B. Aerosile® (Degussa)). Als weitere Zusatzsfoffe seien beispielweise die Kürzfasern auf organischer oder anorganischer Basis (z.B. Glasfasern, Textilfasern) genannt.

Um die Benetzung des Untergrundes zu erhöhen können dem Polyurethan-Elastomer übliche Netzmittel zugegeben werden: beispielsweise Poloxamere (Copolymer von Polyoxyethylene and Polyoxypropylene), Sorbitanester, Fettsäuren wie Span.RTM (Sigma-Aldrich), Ester von Polyoxyethylenesorbitan und Fettsäuren, wie Polysorbate oder Polysorbate.RTM (Spectrum Chemical), Polyoxyethylierte hydrierte Castor Öle wie etwa Cremophor.RTM. (BASF), Polyoxyethylene Stearate, z.B. Myrj.RTM. (Uniqema) oder jede Kombination dieser Netzmittel. Vorzugsweise ist als Netzmittel ein Polysorbat einzusetzen.

Zusätzlich kann das Polyurethan-Elastomer Tackifierharze enthalten. Es können natürliche, modifizierte natürliche und synthetische Harze eingesetzt werden, typischerweise mit einem Molekulargewicht bis zu 1500 g / mol. Die Kompatibilität der Harze mit den weiteren Komponenten ist jeweils in fachüblichen Routineversuchen zu prüfen. Beispielsweise sind Kohlenwasserstoffharze geeignet, insbesondere C5- bis C9-Harze, vorzugsweise mit C5-Harzen modifizierte C9-Harze, und dergleichen. Die gesamten Kohlenwasserstoffharze können teil- oder vollhydriert sein. Ebenfalls zum Einsatz kommen Naturharze wie Balsamharz oder Tallharz. Die genannten Harze können auch verestert werden mit entsprechenden polyfunktionellen Alkoholen wie Pentaerythritester, Glycerinester, Diethylenglykolester, Triethylenglykolester oder Methylester und so eingesetzt werden. Bekannte Handelsprodukte sind z. B. Staybelite ster 10, "Foral" 85-105, "Hercolyn" D, "Alresen" 214 R, "Alresen" 191 R, "Alresen" 500 R 80 und "Cellolyn" 21 s. Polyterpenharze wie auch die Terpenphenolharze können ebenfalls als Tackifierharze einformuliert werden wie auch die synthetischen Harze: Keton-, Kumaron- und Indenharze und auch Kohlenwasserstoff-Harze sind auch möglich z.B. unter Handelsnamen wie "Ketonharz" N, "Lutonal" J 30, "Lutonal" J 60, "Vinnapas" B 17, "Vinnapas" 50 V 1, Kohlenwasserstoffharz 95 KW 10, KW 20 and KW 30. Polyvinylether ist auch ein wirksamer Tackifier. Acrylatharze können ebenfalls alleine oder in Mischungen mit obengenanten Tackifiern eingesetzt werden.

Damit ist eine Wundauflage Gegenstand der vorliegenden Erfindung, die als Wundkontaktschicht ein Polyurethan-Elastomer enthält, welches erhältlich ist durch eine Polymerization von wenigstens einer aliphatischen und/oder cycloaliphatischen Isocyanat-Komponente mit einer Polyetherpolyol-Komponente und welches ein Klebendes Polyurethan-Elastomer oder ein Polyurethan-Elastomer-Haftklebstoff ist. Insbesondere enthält die Wundkontaktschicht klebendes Polyurethan-Elastomer oder ein Polyurethan-Elastomar-Haftklebstoff, welches erhältlich ist durch die Polymerisation von isophorondiisocyanat oder einem modifizierten Isophorondiisocyanat mit wenigstens einer Diol- oder Polyolkomponente, vorzugsweise 2, 3, 4, 5 oder 6 Hydroxylgruppen aufweisenden Polyetherpolyolen mit OH-Zahlen von 20 bis 112 und einem Ethylenoxid (EO)-Gehalt von ≥ 10 Gew.%, vorzugsweise 10 bis 40 Gew.%, besonders bevorzugt 10 bis 20 Gew. %.

Diese Wundkontaktschichten weisen eine besonders gute Wasserdampfdurchlässigkeit auf. Damit ist insbesondere auch eine Wundauflage umfassend eine hydrophile Wundkontaktschicht, wobei die ein hydrophiles Polyurethan-Elastomer umfassende Wundkontaktschicht, die bei einem vollflächigen, unterbruchsfreien Flächenauftrag von 100 g/m$^2$ auf einem Trägermaterial eine Wasserdampfdurchlässigkeit von > 2000 g/ m$^2$/ 24 h, insbesondere von > 2500 g/m$^2$/24 h aufweist Gegenstand der vorliegenden Erfindung, wobei die Wasserdampfdurchlässigkeit gemessen wird nach DIN EN 13726-2: 2002 - MVTR mit Kontakt zu Wasser (vgl. Test 4b).

Weiterhin bevorzugt handelt es sich bei dem hydrophilen Polyurethan-Elastomer um ein wasserfreies Elastomer. Hierbei soll im Zusammenhang mit der vorliegenden Erfindung unter einem wasserfreien Polyurethan-Elastomer oder jeder wasserfreien Verbindung oder Komponente ein Elastomer, eine Verbindung oder Komponente verstanden sein, die/ das weniger als 4 Gew.-% Wasser bezogen auf das Gewicht des/ der jeweiligen Elastomers, Verbindung oder Komponente enthält. Insbesondere enthält ein/ eine solches/ solche Elastomer Verbindung oder Komponente weniger als 2 Gew.-% Wasser, insbesondere weniger als 1 Gew.-% und ganz besonders bevorzugt weniger als 0,5 Gew.-% Wasser

Diese hydrophilen Polyurethan-Elastomere weisen darüber hinaus klebende Eigenschaften auf. Gemäß der vorliegenden Erfindung umfasst die Wundkontaktschicht daher ein klebendes hydrophiles Polyurethan-Elastomer oder einen hydrophilen Polyurethan-Elastomer-Haftklebstoff. Dabei handelt es sich bei dem klebenden Polyurethan-Elastomer oder dem Polyurethan-Elastomer-Haftklebstoff um ein Elastomer, das eine schwache bis mäßig starke Haftklebrigkeit auf menschlicher Haut oder Gewebe aufweist. Insbesondere weist eine erfindungsgemäße Wundauflage eine Klebkraft von 0,02 bis 5 N/25 mm, insbesondere von 0.02 bis 3 N/25 mm und ganz besonders bevorzugt von 0,02 bis 2 N/ 25 mm auf. Hierbei wird die Klebkraft gemäß Test 1 (vgl. Testmethoden) gegen Stahl unter einem Abzugswinkel von 90° gemessen.

Als Wundkontaktschicht ist gemäß der vorliegenden Erfindung eine Schicht zu verstehen, die eine erste und eine zweite Seite aufweist, wobei die erste Seite im anwendungsgerechten Zustand der Wundauflage einen direkten Kontakt zu einer Wunde bildet. Hinsichtlich der Form und Ausmaße der Wundkontaktschicht sind hierbei keine Grenzen gesetzt. Diese Wundkontaktschicht kann in Bezug auf die absorbierende Schicht vollflächig oder diskontinuierlich sein und/ oder eine gleichmäßige oder eine profilierte Schichtdicke aufweisen und/ oder regelmäßige oder unregelmäßige Muster aufweisen.

Gemäß der vorliegenden Erfindung soll die Wundkontaktschicht mit der absorbierenden Schicht verbunden sein. Hiermit soll verstanden sein, dass die Wundkontaktschicht in mindestens einem Bereich ihrer zweiten Seite mit einer ersten, im anwendungsgerechten Zustand zu einer Wunde weisenden Seite der absorbierenden Schicht in direktem Kontakt steht. Besonders bevorzugt umfasst die Wundauflage eine Wundkontaktschicht, deren zweite Seite vollflächig mit der ersten Seite der absorbierenden Lage verbunden ist. Alternativ hierzu kann die Wundkontaktschicht auch integral mit der absorbierenden Schicht verbunden sein. Hiermit soll verstanden sein, dass die beiden miteinander verbundenen und aneinandergrenzenden Schichten an ihren Grenzflächen eine Übergangsschicht bilden, die nicht separiert werden kann. Durch diese integral verbundenen Schichten wird darüber hinaus ein Laminat zur Verfügung gestellt, das keine separierbare Schichten umfasst, die chemisch und/ oder physikalisch miteinander verbunden sind.

Dem entsprechend ist eine mehrschichtige Wundauflage umfassend eine Trägerschicht, eine absorbierende Schicht mit einer ersten und einer zweiten Seite und eine hydrophile Wundkontaktschicht mit einer ersten und einer zweiten Seite, wobei die zweite Seite der Wundkontaktschicht vollflächig mit der ersten Seite der absorbierenden Schicht verbunden ist und ein hydrophiles Polyurethan-Elastomer umfasst, ebenfalls Gegenstand der vorliegenden Erfindung. Besonders bevorzugt weist diese Wundauflage eine Wundkontaktschicht auf, die in Bezug auf die erste Seite der absorbierenden Schicht vollflächig ist. Ganz besonders bevorzugt weist diese Wundauflage eine Wundkontaktschicht auf, die integral mit der absorbierenden Schicht verbunden ist und die insbesondere vollflächig in Bezug auf die erste Seite der absorbierenden Schicht ist.

Schließlich kann auch vorgesehen sein, dass die Wundauflage eine Wundkontaktschicht aufweist, die in Bezug auf die erste Seite der absorbierenden Schicht nicht vollflächig ist, sondern einzelne Bereiche der absorbierenden Schicht ausspart, beispielsweise um hier einen Klebeauftrag zur Befestigung der Wundauflage auf der Haut eines Patienten anzubringen. Alternativ kann die Wundauflage auch nicht vollflächig in Bezug auf die absorbierende Schicht ausgestaltet sein, indem eine diskontinuierliche Wundkontaktschicht gewählt wird, die regelmäßige oder unregelmäßig angeordnete Auspaarungen vorsieht. Diese Aussparungen stellen einen verbesserten Durchgang von Wundflüssigkeiten von der Wunde zur absorbierenden Schicht zur Verfügung.

Als besonders vorteilhafte Ausführungen haben sich weiterhin Wundauflagen gezeigt, die eine Wundkontaktschicht aufweisen, deren Schichtdicke weniger als 1000 μm aufweist. Insbesondere weist damit eine erfindungsgemäße Wundauflage eine Wundkontaktschicht mit einer Schichtdicke von 10 bis 1000 μm, insbesondere von 10 bis 500 μm und ganz besonders bevorzugt von 10 bis 250 μm auf. Wundauflagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Der Ausdruck "hydrophil", wie er im Zusammenhang mit der vorliegenden Erfindung verwendet wird, beschreibt Elastomere oder Oberflächen von Lagen, die durch auf diese Oberflächen oder Elastomere abgesetzten, wässrigen Flüssigkeiten (beispielsweise wässrige Körperflüssigkeiten wie Wundsekret) benetzbar sind. Hydrophilie und Benetzbarkeit können über einen Kontaktwinkel und der Oberflächenspannung der beteiligten Flüssigkeiten und Feststoffe definiert werden. Demnach soll im Zusammenhang mit der vorliegenden Erfindung unter einer hydrophilen Schicht oder einem hydrophilen Polyurethan-Elastomer eine solche Schicht oder ein solches Elastomer verstanden sein, die/ das gegenüber Wasser einen Kontaktwinkel kleiner als 90° aufweist oder wenn das Wasser zum spontanen Spreiten auf der Oberfläche der Schicht oder des Elastomers tendiert, wobei die Bestimmung des Kontaktwinkels analog DIN EN 828 durchgeführt wird. Beide Vorgänge sind in der Regel koexistent. Dem gegenüber wird eine Schicht oder ein Elastomer als hydrophob bezeichnet, wenn ein Kontaktwinkel von größer oder gleich 90° ausgebildet wird und kein Spreiten beobachtet wird.

Demnach umfasst eine erfindungsgemäße Wundauflage ein hydrophiles Polyurethan-Elastomer oder eine hydrophile Wundkardsktschicht, das/die einen Kontaktwinkel gegenüber Wasser von weniger als 90°, insbesondere von weniger als 75° und ganz besonders von weniger als 65° aufweist, wobei der Kontaktwinkel analog DIN EN 828 ermittelt wird.

Darüber hinaus sind gemäß der vorliegenden Erfindung solche Polyurethan-Elastomere bevorzugt, die selbst absorbierende Eigenschaften aufweisen. Insbesondere sind dabei Polyurethan-Elastomere bevorzugt, die mindestens 50 % ihres Eigengewichtes an wässrigen Flüssigkeiten aufnehmen können. Damit ist eine mehrschichtige Wundauflage umfassend eine Trägerschicht, eine absorbierende Schicht und eine hydrophile Wundkontaktschicht, wobei die Wundkontaktschicht mit der absorbierenden Schicht verbunden ist und ein hydrophiles Polyurethan-Elastomer umfasst, das mindestens 50 Gew.-% bezogen auf sein Eigengewicht an Salzlösung absorbiert, ebenfalls Gegenstand der vorliegenden Erfindung. Insbesondere absorbiert ein solches Polyurethan-Elastomer 50 bis 200 Gew.-% und ganz besonders bevorzugt 50 bis 150 Gew.-% an Salzlösung bezogen auf sein Eigengewicht, wobei eine Bestimmung der Absorptionsleistung analog DIN-EN 13726-1 (2002) durchgeführt wird.

Als absorbierende Schicht kann jedes heute in der modernen Wundbehandlung übliches, als absorbierende Schicht einzusetzendes Material Verwendung finden. Hierbei sind insbesondere solche Materialien zu nennen, die in der feuchten Wundtherapie angewendet werden können. Ganz besonders sind jedoch solche absorbierende Schichten bevorzugt, die sowohl Wundsekret aufnehmen und somit absorbierend wirken als auch Feuchtigkeit an die Wunde abgeben. Weiterhin bevorzugt sind solche absorbierende Schichten, die transparent oder transluzent sind. Gemäß einer besonders bevorzugten Ausgestaltung der vorliegenden Erfindung umfasst die Wundauflage als absorbierende Schicht einen hydrophilen Polymer-Schaum, ein absorbierendes Vlies oder Nonwoven, eine mindestens ein Hydrocolloid umfassende Polymermatrix, einen gefriergetrockneten Schaum oder Kombinationen hiervon.

Wird als absorbierende Schicht ein hydrophiler Polymerschaum verwendet, so hat sich gezeigt, dass sich die Wundauflage besonders gut an eine zu behandelnde Wunde anpasst.

Als Polymerschäume sind hierbei insbesondere hydrophile Polyurethan-Schäume geeignet. Dem gemäß umfasst eine besonders bevorzugte Wundauflage eine absorbierende Schicht aus einem hydrophilen Polyurethan-Schaum. Diese Polyurethan-Schäume weisen eine freie Absorption von mindestens 10 g/ g, insbesondere mindestens 12 g/g und ganz besonders bevorzugt von mindestens 15 g/ g auf, wobei die freie Absorption gemäß der DIN-EN 13726-1 (2002) bestimmt wird. Weiterhin bevorzugt weisen diese Schäume eine Porengröße von durchschnittlich weniger als 1000 $\mu$m, insbesondere 200 bis 1000 $\mu$m und ganz besonders bevorzugt 200 bis 700 $\mu$m auf. Hierbei kann vorgesehen sein, dass die Porengröße an einer ersten Oberfläche der absorbierenden Schicht im Wert verschieden ist von der Porengröße einer zweiten Oberfläche der absorbierenden Schicht. Weiterhin bevorzugte hydrophile, Polyurethan-Schäume weisen eine Dichte von weniger als.150 kg/m$^3$, insbesondere weniger als 140 kg/m$^3$ und ganz besonders bevorzugt 70 bis 120 kg/m$^3$ auf.

In einer erfindungsgemäßen Alternative umfasst die absorbierende Schicht insbesondere wasserunlösliche Fasern aus Cellulose, insbesondere weitgehend delignifizierte technische Zellstofffasern, insbesondere Holzzellstofffasern, insbesondere einer Faserlänge von < 5mm Verwendung finden. Das Fasermaterial kann auch hydrophiles Fasermaterial aus regenerierter Cellulose, Carboxymethylcellulose, Carboxyethylcellulose, Hydoxymethylcellulose oder Hydroxyethylcellulose enthalten. Es kann auch vorgesehen sein, eine Fasermischung aus Cellulose-, regenerierter Cellulose-, Carboxymethylcellulose-, Carboxyethylcellulose-, Hydoxymethylcellulose- oder Hydroxyethylcellulose-Fasern und Fasern aus Polyethylen, Polypropylen oder Polyester vorzusehen. In einer ganz besonders bevorzugten Ausführungsform umfasst die absorbierende Schicht eine Mischung aus Cellulosefasern, Polypropylenfasern und partikelförmigem superabsorbierendem Polymer vorzugsweise aus vernetztem Natrium-Polyacrylat.

Eine weitere alternative erfindungsgemäße Wundauflage umfasst eine absorbierende Schicht, die aus einer hydrophoben Matrix besteht, in die Hydrocolloide dispergiert sind. Gemäß der vorliegenden Erfindung soll unter einem Hydrocolloid ein Material verstanden sein, das ein hydrophiles, synthetisches oder natürliches Polymermaterial ist, das löslich oder absorbierend und/ oder quellend in Wasser ist. Vorzugsweise umfasst eine absorbierende Schicht ein Hydrocolloid aus einem synthetischen oder natürlichen Polymermaterial, das ausgewählt wird aus der Gruppe Alginsäure und deren Salze sowie deren Derivate, Chitin oder dessen Derivate, Chitosan oder dessen Derivate, Pektin, Cellulose oder dessen Derivate wie Celluloseether oder Celluloseester, vernetzte oder nicht vernetzte Carboxyalkylcellulose oder Hydroxyalkylcellulose, Polyvinylalkohol, Polyvinylpyrrolidon, Agar, Guargum oder Gelatine.

Das Hydrocolloid kann hierbei sowohl in Form von Fasern als auch in Form von Partikeln und/oder Fasern innerhalb der Matrix vorliegen. Insbesondere kann das Hydrocolloid in Form von Partikeln in einer klebenden Polymermatrix vorliegen. Die klebende Polymermatrix umfasst dabei mindestens ein nicht hydriertes, teilhydriertes oder vollhydriertes Co-Blockpolymer ausgewählt aus der Gruppe der AB-Diblock-Copolymere und/oder ABA-Triblock-Copolymere, das aus den Monomeren Styrol, Butadien und/oder Isopren aufgebaut ist. Der Anteil an Hydrocolloidpartikel in der Wundkontaktschicht kann vorzugsweise 10 bis 70 Gew.-% bezogen auf das Gesamtgewicht der absorbierenden Schicht aufweisen. Eine solche Zusammensetzung ist zum Beispiel mit der EP 1 007 597 B1 bekannt.

Als Trägerschicht können verschiedene Materialien eingesetzt werden. Üblicherweise werden in Wundauflagen textile Trägermaterialien, Nonwoven, Polymerfilme oder Polymerschäume eingesetzt. Als Trägerschicht einer erfindungsgemäßen Wundauflage können insbesondere Polymerfilme oder Polymerschäume eingesetzt werden. Ganz besonders bevorzugt sind Polymerfilme, die eine hohe Wasserdampfdurchlässigkeit aufweisen. Hierzu sind besonders Filme geeignet, die aus Polyurethan, Polyetherurethan, Polyesterurethan, Polyether-Polyamid-Copolymeren, Polyacrylat oder Polymethacrylat gefertigt werden. Insbesondere ist als Polymerfilm ein Polyurethanfilm, Polyesterurethanfilm oder Polyetherurethanfilm bevorzugt. Ganz besonders sind aber auch solche Polymerfilme bevorzugt, die eine Dicke von 15 bis 50 $\mu$m, insbesondere 20 bis 40 $\mu$m und ganz besonders bevorzugt von 25 bis 30 $\mu$m aufweisen. Die Wasserdampfdurchlässigkeit des Polymerfilms der Wundauflage weist vorzugsweise mindestens 750 g/ m$^2$/24 Std., insbesondere mindestens 1000 g/ m2/ 24 Std. und ganz besonders bevorzugt mindestens 2000 g/ m$^2$/ 24 Std. auf (gemessen nach DIN EN 13726). In besonders bevorzugten Ausführungsformen weisen diese Filme einen feuchtigkeitsdichten, klebenden Randabschnitt auf. Dieser Randabschnitt gewährleistet, dass die Wundauflage an seinem bestimmungsgemäßen Ort appliziert und fixiert werden kann. Darüber hinaus ist sichergestellt, dass keine Flüssigkeit zwischen der Folie und der die zu behandelnden Fläche umgebenden Haut austreten kann. Als besonders bevorzugt sind solche Klebstoffe zu betrachten, die in einem dünnen Auftrag von 20 bis 35 g/ m$^2$ zusammen mit dem Film eine Wasserdampfdurchlässigkeit von mindestens 800 g/ m$^2$/24 Std. und vorzugsweise von mindestens 1000 g/ m$^2$/24 Std. (gemessen nach DIN EN 13726) aufweisen.

In einer alternativen und besonders bevorzugten Ausführung einer erfindungsgemäßen Wundauflage kann die Trägerschicht auch aus einem Polymerschaum gefertigt sein. Insbesondere ist in dieser Ausgestaltung vorgesehen, dass der Polymerschaum ein Polyurethan-Schaum ist. Diese Polyurethanschäume sind aus im Wesentlichen geschlossenporigen Polyurethanschäumen gefertigt und weisen insbesondere eine Dicke von 0,01 bis 2 mm auf. Dabei zeigen sich im Wesentlichen geschlossenporige Polyurethanschäume als besonders vorteilhaft, da diese Schäume sowohl wasserdampfdurchlässig sind als auch eine Barriere für Schmutz und Keime darstellen. Dieses Trägematerials weist besonders bevorzugt eine Wasserdampfdurchlässigkeit von mindestens 750 g/ m$^2$/ 24 Std., insbesondere mindestens 1000 g/m$^2$/ 24 Std. und ganz besonders bevorzugt mindestens 1200 g/m$^2$/ 24 Std. auf (gemessen nach DIN EN 13726) auf.

In einer weiterhin bevorzugten Ausführung weist die erfindungsgemäße Wundauflage als Trägerschicht und als absorbierende Schicht ein Laminat aus zwei verschiedenen Polyurethan-Schäumen auf. Insbesondere besteht diese Wundauflage aus einem Laminat aus zwei verschiedenen Polyurethan-Schäumen, das auf der im anwendungsgerechten Zustand der Wundauflage zur Wunde weisenden Seite mit einer hydrophilen Wundkontaktschicht aus einem klebenden, hydrophilen Polyurethan-Elastomer beschichtet ist. Damit ist insbesondere eine Wundauflage bestehend aus einer Trägerschicht aus einem ersten hydrophoben, für Wasserdampf durchlässigen Polyurethan-Schaum, einer absorbierenden Schicht aus einem hydrophilen, absorbierenden Polyurethan-Schaum und einer mit der absorbierenden Schicht verbundenen, insbesondere integral verbundenen hydrophilen Wundkontaktschicht, die ein von dem hydrophilen Schaum verschiedenes, klebendes, hydrophiles Polyurethan-Elastomer umfasst, Gegenstand der vorliegenden Erfindung. Insbesondere umfasst diese Wundauflage auch eine absorbierende Schicht, die eine höhere freie Absorption aufweist als die Wundkontaktschicht.

Diese Wundauflage ist besonders vorteilhaft als unterstützende Maßnahme in der modernen Wundbehandlung einzusetzen, da durch die Wundkontaktschicht keine Wundverklebung stattfindet, keine Mazeration der die Wunde umgebenden Haut stattfindet, eine schnelle Aufnahme von der Wunde abgegebenen Wundflüssigkeiten stattfindet und eine Fixierung der Wundauflage auf der die Wunde umgebenden Haut vorgenommen werden kann.

Des Weiteren hat sich herausgestellt, dass eine erfindungsgemäße Wundauflage in Abhängigkeit von der Art der Wunde auch über einen Zeitraum von bis zu sieben Tagen verwendet werden kann. Dies ist möglich, da die Wundauflage besonders leistungsfähig hinsichtlich ihrer Absorptionskapazität ist. Insbesondere weist eine erfindungsgemäße Wundauflage eine maximale Flüssigkeitsaufnahmekapazitat (max. Fl.) von mindestens 5000 g/m$^2$/24 h und weiterhin bevorzugt von 5000 bis 10000 g/m$^2$/ 24 h aufweist, wobei die maximale Flüssigkeitsaufnahmekapazität bestimmt wird durch folgende Formel:

$$\max. Fl. = (Flächengewicht \ x \ Flüssigkeitsaufnahme) + MVTR \left[\frac{g}{m^2 \, 24h}\right]$$

Hierin bedeutet:

- Flächengewicht: Flächengewicht der Probe in g/m$^2$
- Flüssigkeitsaufnahme: Flüssigkeitsaufnahme nach Test 3 (vgl. Testmethoden)
- MVTR: Wasserdampfdurchlässigkeit nach Test 4 (vgl. Testmethoden)

In einer alternativen Form einer erfindungsgemäßen Wundauflage umfasst eine Wundkontaktschicht weiterhin mindestens ein die Wundheilung aktiv unterstützendes Mittel, insbesondere ein antimikrobielles Mittel, ein Vitamin oder Provitamin, eine Fettsäure oder Fettsäureester oder ein den Gewebeaufbau aktiv förderndes Mittel.

Gemäß einer besonders bevorzugten Alternative umfasst die Wundkontaktschicht weiterhin mindestens ein antimikrobielles Mittel. Insbesondere sind hierbei antimikrobielle Metalle oder deren Salze, insbesondere Silber oder dessen Salze geeignet. In einer besonders bevorzugten Ausführung umfasst die Wundkontaktschicht ein antimikrobielles Mittel und ein Trägermaterial für das antimikrobielle Mittel. Hierbei können bevorzugt als Trägermaterial ein Nonwoven oder ein textiles Material wie Gestrick, Gewirk oder Gewebe verwendet werden, das mit einem antimikrobiell wirkenden Metall vorzugsweise Silber oder Silbersalze beschichtet ist. Dabei ist es besonders vorteilhaft, wenn das hydrophile Polyurethan-Elastomer wasserfrei ist.

Gemäß einem weiterführenden Gedanken der vorliegenden Erfindung, ist auch eine mehrschichtige Wundauflage umfassend eine Trägerschicht, eine absorbierende Schicht, eine hydrophile Wundkontaktschicht und eine Verteilerschicht, wobei die Wundkontaktschicht ein hydrophiles Polyurethan-Elastomer umfasst, ebenfalls Gegenstand der vorliegenden Erfindung. Insbesondere ist die absorbierende Schicht mit der Wundkontaktschicht verbunden. Eine solche Wundauflage weist besonders vorteilhaft eine Verteilerschicht zwischen der Trägerschicht und der absorbierenden Schicht auf, die insbesondere aus einem hydrophilen Polyurethan-Schaum besteht. Durch die Verteilerschicht wird erreicht, dass eine Verteilung der aufgenommen Wundflüssigkeiten über die gesamte Fläche der Wundauflage insbesondere oberhalb der absorbierenden Schicht ermöglicht wird, das heißt das die Aufnahme der Wundflüssigkeiten nicht nur in z-Richtung (von der Wunde weg in Richtung Trägerschicht), sondern auch in x-y-Richtung (über die Fläche der Wundauflage) erfolgt

Als weiterhin vorteilhaft hat sich herausgestellt, dass die Wundauflage mindestens eine Verteilerschicht $V_n$ zwischen der absorbierenden Schicht und der Trägerschicht umfasst, wobei n = 1 bis 4 bedeutet und $V_n$ mit n = 1 die der Haut oder der Wunde am nächsten liegende Schicht und $V_n$ mit n = 4 die der Haut oder der Wunde am weitesten entfernte Schicht bedeutet. Dies gewährleistet eine besonders gleichmäßige Verteilung der aufgenommenen Flüssigkeiten innerhalb der Wundauflage und beabstandet von der Wunde oberhalb der Haut oder der Wunde. Insbesondere hat sich herausgestellt, dass eine Verteilerschicht $V_n$ mit n = 1 ausgebildet als ein Tissue, Nonwoven, Gewebe, Gestrick und/oder Gewirk besonders leistungsfähig ist.

Zusätzlich zu der Verteilerschicht $V_n$ kann die Wundauflage mindestens eine weitere absorbierende Schicht $A_m$ zwischen einer ersten Verteilerschicht $V_1$ und der Trägerschicht umfassen, wobei m = 1 bis 3 bedeutet und $A_m$ mit m = 1 die der Haut oder der Wunde am nächsten liegende Schicht und Am mit m = 3 die der Haut oder der Wunde am weitesten entfernt liegende Schicht bedeutet. Dabei kann die Wundauflage, die mindestens eine Verteilerschicht aufweist, ein Nonwoven aus einem hydrophilen Fasermaterial aufweisen und ein gelbildendes Superabsorbermaterial umfassen. Hierbei kann als hydrophiles Fasermaterial insbesondere wasserunlösliche Fasern aus Cellulose, insbesondere weitgehend delignifizierte technische Zellstofffasern, insbesondere Holzzellstofffasern, insbesondere einer Faserlänge von < 5mm Verwendung finden. Das Fasermaterial kann auch hydrophiles Fasermaterial aus regenerierter Cellulose, Carboxymethylcellulose, Carboxyethylcellulose, Hydoxymethylcellulose oder Hydroxyethylcellulose enthalten.

Gemäß einem weiterführenden Gedanken ist auch die Verwendung einer hydrophilen Wundkontaktschicht umfassend ein hydrophiles Polyurethan-Elastomer zur Herstellung eines Mittels zur aktiven oder passiven Unterstützung des Gewebeaufbaus in Wunden insbesondere in chronischen Wunden offenbart. Insbesondere ist hierbei ein Verbund aus einer direkt mit dieser hydrophilen Wundkontaktschicht verbundenen absorbierenden Schicht hierzu zu verwenden. Ganz besonders geeignet ist eine Wundauflage gemäß der vorliegenden Erfindung.

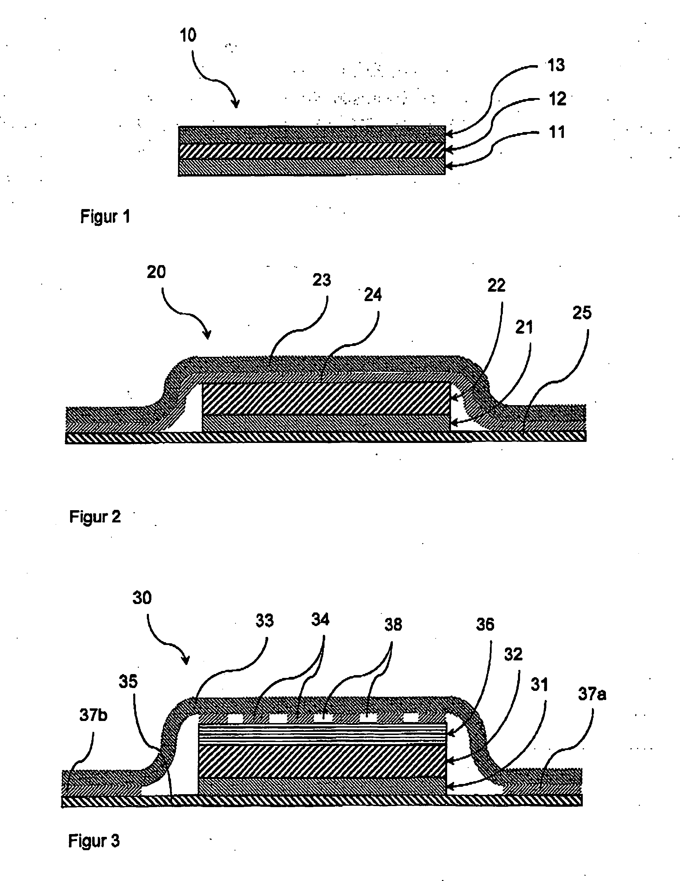

Im Folgenden soll die Erfindung an Hand von Zeichnungen erläutert werden, ohne dass diese Zeichnungen eine bezüglich der Erfindung einschränkende Wirkung haben sollen. Dabei sind mit Figur 1 - 6 jeweils alternative Ausführungen einer erfindungsgemäßen Wundauflage im Querschnitt gezeigt.

Mit Figur 1 ist der einfachste Aufbau einer erfindungsgemäßen Wundauflage (10) wiedergegeben. Die Wundauflage besteht aus einer Wundkontaktschicht (11) aus einem hydrophilen Polyurethan-Elastomer, die auf einen absorbierenden Polyurethan-Schaum als absorbierende Schicht (12) aufgebracht und mit diesem integral verbunden ist. Als Trägerschicht (13) weist diese Wundauflage einen hydrophoben, für Wasserdampf durchlässigen Polyurethan-Schaum auf. Diese Trägerschicht ist für Wasser und Keime undurchlässig und weist geschlossene Poren auf. In diesem Wundverband dient die Wundkontaktschicht als nicht-wundverklebende Abstandsschicht, die durch ihre Hydrophilie ein schnelles Aufnehmen von Wundflüssigkeit ermöglicht.

Mit Figur 2 ist eine erfindungsgemäße Wundauflage (20) als so genannter Inselverband gezeigt. Die Wundauflage besteht aus einem absorbierenden, hydrophilen Polyurethanschaum als absorbierende Lage (22), die auf der im anwendungsgerechten Zustand einer Wunde zugewandten Seite mit einer hydrophilen Wundkontaktschicht aus einem klebenden hydrophilen Polyurethan-Elastomer (21) vollflächig beschichtet ist. Auf der im anwendungsgerechten Zustand der Wunde abgewandten Seite der absorbierenden Lage ist mittels eines vollflächig aufgebrachten Acrylat-Haftklebers (24) eine Trägerschicht (23) aus einem geschlossenzelligen Polyurethan-Schaum aufgebracht. Sowohl die Wundkontaktschicht (21) als auch der die Wundkontaktschicht allseits umgebende klebende Rand sind vor Gebrauch der Wundauflage mit einem siliconisierten Release-Papier (25) abgedeckt. Diese Wundauflage weist zwei verschieden stark haftende Haftklebezonen auf, wobei die Wundkontaktschicht eine Klebkraft von 0,10 N/ 25 mm (gemessen gegen Stahl, vgl. Test 1) aufweist.

Mit Figur 3 ist ebenfalls eine erfindungsgemäße Wundauflage (30) als Inselverband gezeigt. Die Wundauflage besteht aus einer hydrophilen Wundkontaktschicht aus einem hydrophilen Polyurethan-Elastomer (31), das vollflächig auf einen hydrophilen Polyurethan-Schaum (32) aufgebracht und mit dem Schaum integral verbunden ist. Die absorbierende Schicht weist eine Schichtdicke von 5 mm auf, wobei der Polyurethan-Schaum eine Porengröße von 300 bis 900 $\mu$m aufweist Auf der der Wundkontaktschicht gegenüberliegenden Oberfläche der absorbierenden Schicht ist zwischen der Trägerschicht (33) und der absorbierenden Schicht (32) eine weitere absorbierende Lage (36) aufgebracht. Diese absorbierende Lage (36) dient als Verteilerschicht für bereits durch die Wundkontaktschicht (31) und die absorbierende Schicht (32) aufgenommene Flüssigkeitsmengen. Die absorbierende Verteilerschicht ermöglicht eine gleichmäßige Verteilung der aufgenommenen Flüssigkeiten in x-y-Richtung, wogegen die absorbierende Schicht (32) als auch die Wundkontaktschicht (31) eine Absorption der Wundflüssigkeiten in z-Richtung, d.h. senkrecht zur Wundoberfäche gewährleisten. Die Trägerschicht besteht aus einem dünnen für Wasserdampf sehr gut durchlässigen Polyurethan-Film, der eine Schichtdicke von 70 $\mu$m aufweist. Die Verteilerschicht besteht aus einem Verteilervlies aus Cellulosefasern, das mittels streifenförmig aufgebrachten Acrylat-Haftklebstoff (34) an der Trägerschicht (33) befestigt ist. Somit kann durch die von Acrylat-Klebstoff frei bleibenden Stellen (38) ein gegenüber einem vollflächig beschichteten Film verbesserter Wasserdampfaustausch mit der Umgebung stattfinden. Sowohl die Wundkontaktschicht (31) als auch der die Wundkontaktschicht allseits umgebende klebende Rand (37a, 37b) aus einem Acrylat-Haftklebstoff sind vor Gebrauch der Wundauflage mit einem siliconisierten Release-Papier (35) abgedeckt.

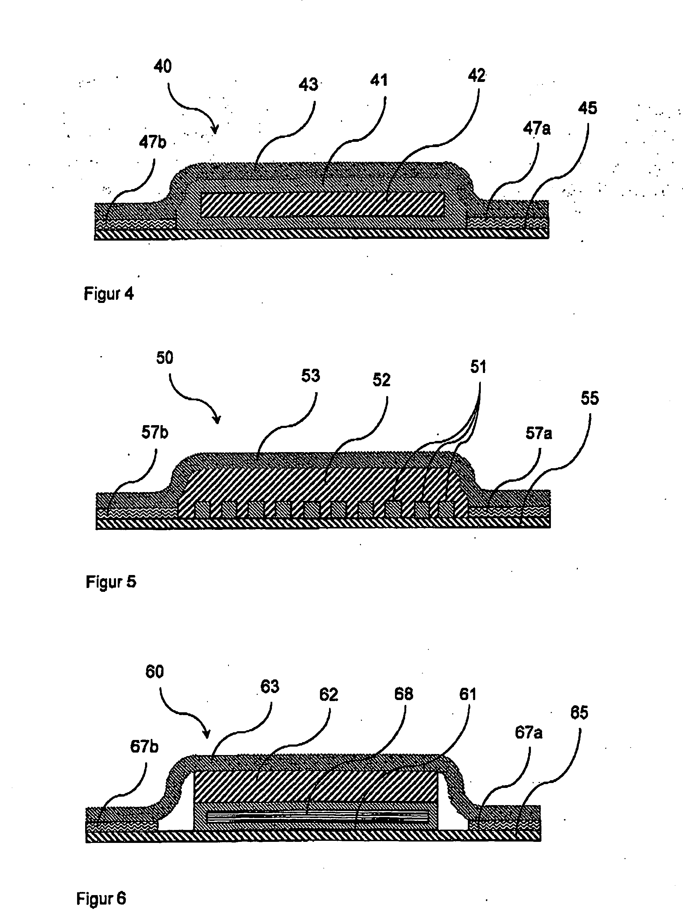

Mit Figur 4 ist eine alternative Gestaltung einer erfindungsgemäßen Wundauflage (40) gezeigt. In dieser Ausführung ist eine absorbierender Lage (42) aus einem hydrophilen Polyurethan-Schaum allseitig von einem klebenden, hydrophilen Polyurethan-Elastomer (41) umgeben. Das Elastomer bildet somit die Wundkontaktschicht als auch eine Verbindung zwischen der Trägerschicht (43) und der absorbierenden Lage (42). Die Tägerschicht besteht aus einem für Wasserdampf durchlässigen, geschlossenzelligen Polyurethanschaum. Sowohl das hydrophile Polyurethan-Elastomer (41) als auch der die Wundkontaktschicht allseits umgebende klebende Rand (47a, 47b) aus einem Acrylat-Haftklebstoff sind vor Gebrauch der Wundauflage mit einem siliconisierten Release-Papier (45) abgedeckt.

Mit Figur 5 ist eine erfindungsgemäße Wundauflage (50) gezeigt, die ein direkt verbundenes Laminat aus einer Trägerschicht (53) und einer absorbierenden Lage (52) aus zwei verschiedenen Polyurethanschäumen aufweist. Die Trägerschicht besteht aus einem geschlossenzelligen, für Wasserdampf durchlässigen Polyurethan-Schaum, der an den Rändern (57a, 57b) mit einem Acrylat-Haftklebstoff beschichtet ist, wogegen die absorbierende Schicht aus einem hydrophilen Polyurethan-Schaum besteht. In der absorbierenden Lage sind auf der im anwendungsgerechten Zustand zur Wunde gerichteten Oberfläche der absorbierenden Lage (52) streifenförmige Ausnehmungen vorgesehen, in die ein hydrophiles Polyurethan-Elastomer (51) eingefüllt ist. Alternativ (hier nicht dargestellt) kann auch vorgesehen sein, dass das hydrophile Polyurthan-Elastomer in Mustern auf eine ebene Oberfläche einer absorbierenden Lage (52) aufgebracht ist. In jedem Fall weist somit die Wundauflage (50) eine diskontinuierliche, in Mustern aufgebrachte Wundkontaktschicht auf. Für eine geschützte Lagerung ist die im anwendungsgerechten Zustand der Wundauflage zur Wunde oder zu der die Wunde umgebenden Haut weisende Oberfläche der Wundauflage vor ihrem Gebrauch mit einer Polyethylen-Releasefolie (55) abgedeckt.

Mit Figur 6 ist eine alternative erfindungsgemäße Wundauflage (60) als Inseldressing gezeigt. Diese Wundauflage besteht aus einer absorbierenden hydrophilen Polyurethan-Schaumschicht (62), auf die eine Wundkontaktschicht (61) aus einem klebenden, hydrophilen Polyurethan-Elastomer aufgebracht ist. In die Wundkontaktschicht ist ein mit Silber beschichtetes Trägermaterial (68) eingebettet. Das hydrophile Polyurethan-Elastomer ummantelt vollständig das als Netz ausgestaltete Trägermaterial, das seinerseits allseitig mit Silber beschichtet ist. Dadurch, dass das hydrophile Polyurethan-Elastomer selbst absorbierende Eigenschaft aufweist, kann durch die von der Wundkontaktschicht aufgenommene Flüssigkeit Silbermaterial aus dem Verbund ausgelöst und somit eine antimikrobielle Wundkontaktschicht bereit gestellt werden. Darüber hinaus kann aufgrund der klenenden Eigenschaften de Polyurethan-Elastomers ein sicherer Verbund zwischen der absorbierenden Schicht und dem Netz erfolgen. Als Schutz vor Verschmutzung und Keimdurchtritt von außen weist die Wundauflage weiterhin eine Trägerschicht (63) aus einem hydrophoben Polyurethan-Schaum auf. Im Randbereich der Trägerschicht, der die gesamte Wundkontaktschicht umgibt, ist zur Befestigung auf der Haut eines Patienten ein Acrylat-Haftklebsöff (67a, 67b) aufgebracht. Für eine geschützte Lagerung ist die Wundauflage in eine sterile Verpackung eingebracht (hier nicht dargestellt) und die gesamte, im anwendungsgerechten Zustand zur Wunde weisende Seite der Wundauflage vor ihrem Gebrauch mit einer Polyethylen-Releasefolie (65) abgedeckt.

Hervorzuheben ist an dieser Stelle, dass die hier aufgeführten Merkmale der alternativen oder bevorzugten Ausgestaltungen der Erfindungen nicht auf die einzelnen Alternativen zu beschränken sind. Im Zusammenhang mit der vorliegenden Erfindung ist es vielmehr der Fall, dass eine Kombination von Ausgestaltungen bzw. eine Kombination jedes Einzelmerkmals einer alternativen Form mit Merkmalen einer anderen alternativen Ausgestaltung ebenso als erfindungsgemäßer Gegenstand zu sehen ist.

Ausführungsbeispiele

A) Testmethoden:

1) Klebkraft auf Stahl (90 ° Abzugswinkel) in Anlehnung an AFERA 5001) -Test 1

Das zu prüfende Muster wird vor der Prüfung 24 h im Normklima (23 °C, 50-% relative Feuchte) gelagert und danach 3 Proben von je 25 mm Breite und 100 mm Länge entnommen. Der Prüfkörper wird mit der Hand vorsichtig und dehnungsfrei auf Stahlplatten (nach DIN EN 1939) auflaminiert, wobei Luftblesenbildung vermieden wird. Auf die nicht klebende Oberseite des Musters wird ein handelsübliches Verstarkungsklebeband, das nicht dehnbar ist (z.B. Tesa 4104) aufgebracht, um die Dehnung des Schaums zu eliminieren. Der Prüfkörper wird mit Hilfe eines Tape Applicators D 427/1 der Firma Sondes Place Research Institute, Surrey England mit 20 N/ cm definiert angerollt. Die so präparierte Stahlplatte wird in die 90° Abzugsvorrichtung der Zug-Dehnungsmaschine Z-005 der Firma Zwick-Roell, Ulm Deutschland, eingelegt und das freie, über das Muster hinaus stehende Ende des Verstärkungsklebebandes in die obere Klemme eingespannt. Bei einer konstanten Abzugsgeschwindigkeit von 300 mm/ min wird der Kraftverlauf gemessen, der benötigt wird, um die Probe von der Stahlplatte zu lösen. Die Ermittlung der Klebekraft erfolgt durch ein geeignetes PC-Programm nach DIN 53 539 (Verfahren C).

2) Klebkraft auf Stahl (180 ° Abzugswinkel) in Anlehnung an AFERA 5001 - Test 2

Das zu prüfende Muster wird vor der Prüfung 24 h im Normklima (23°C, 50-% relative Feuchte) gelagert und danach 3 Proben von je 25 mm Breite und 100 mm Länge entnommen. Der Prüfkörper wird mit der Hand vorsichtig und dehnungsfrei auf aufgerauten Stahlplatten (bezogen von der Firma Scapa Medical Bedfordshire UK) auflaminiert, wobei Luftblasenbildung vermieden wird. Auf die nicht klebende Oberseite des Musters wird ein handelsübliches Verstärkungsklebeband, das nicht dehnbar ist (z.B. Tesa 4104) aufgebracht, um die Dehnung des Schaums zu eliminieren. Der Prüfkörper wird mit Hilfe eines Anrollers mit einem Gewicht von 3 kg mit einer Geschwindigkeit von ca. 1 cm/ s manuell angerollt. Die so präparierte Stahlplatte wird in die 180° Abzugsvorrichtung der Zug Dehnungsmaschine Z-005 der Firma Zwick-Roell, Ulm Deutschland, eingelegt und das freie, über das Muster hinaus stehende Ende des Verstärkungsklebebandes in die obere Klemme eingespannt. Bei einer konstanten Abzugsgeschwindigkeit von 300 mm/min wird der Kraftverlauf gemessen. Die Ermittlung der Klebekraft erfolgt durch ein geeignetes PC-Programm nach DIN ISO 6133 (Verfahren C).

3) Freie Absorption von Kochsalzlösung - Test 3

Die freie Absorption von physiologischer Natriumchlorid-Lösung (0,9 Gew.-%-ig, NaCl in Wasser) wird in Anlehnung an DIN EN 13726-1 (2002) bestimmt. Der einzige Unterschied zur DIN EN 13726-1 (2002) ist die Verwendung von NaCl-Lösung anstelle von NaCl- und $CaCl_2$-Lösung, wobei folgendes gilt:

$$Flüssigkeitsaufnahme = \frac{M_2 - M_1}{M_1} [g/g] \tag{1}$$

M2 = Nassgewicht der Probe in g
M1 = Trockengewicht der Probe in g

4a) Wasserdampfpenetration (MVTR, bei Kontakt mit Wasserdampf) - Test 4a

Die Wasserdampfpenetration wird in Anlehnung an DIN EN 13726-2 (2002) bestimmt. Eine kreisförmige Probe wird derart aus der Wundauflage ausgeschnitten, dass sie so über der Öffnung der Prüfapparatur festgeklemmt werden kann, dass kein Flüssigkeitsaustritt erfolgt. Die Probe wird derart auf die Prüfapparatur befestigt, dass die Wundkontaktschicht direkten Kontakt mit dem sich bildenden Wasserdampf steht. Vorher ist dieser Zylinder mit 20 ml destilliertem Wasser gefüllt worden. Der Zylinder wird dann für 24 h einem kontrollierten Klima von 37 °C und einer relativen Luftfeuchte < 20 % ausgesetzt. Nach Ablauf der Testzeit erfolgt eine Differenzwägung.

$$MVTR = \frac{M_1 - M_2 \cdot 10000}{A} \left[\frac{g}{m^2 24h}\right] \quad (2)$$

MVTR = moisture vapor transition rate = Wasserdampfdurchlässigkeit
M1 = Ausgangsgewicht in g
M2 = Endgewicht in g
A = Flache der Zylinderbohrung des Prüfgefäßes in cm$^2$

4b) Wasserdampfpenetration (MVTR, bei Kontakt mit Wasser) - Test 4b Messung erfolgt gemäß Messung 4a mit dem einzigen Unterschied, dass die Prüfapparatur umgewendet benutzt wird, so dass die Wundkontaktschicht im direkten Kontakt mit der Wassersäule steht.

5) Bestimmung der maximalen Flüssigkeitsaufnahmekapazität Test 5
Die maximale Flüssigkeitsaufnahmekapazität (max. Fl.) stellt eine zusammengesetzte Größe dar, die sich aus der Wasserdampfdurchlässigkeit gemäß Test 4a, dem Flächengewicht der Probe in g/ m$^2$ sowie der Flüssigkeitsaufnahme gemäß Test 3 ergibt. Hierbei wird die freie Absorption gemäß Test 3 mit der freien Absorption in 24h gleichgesetzt. Die Größe gibt die Absorptionsleistung der Wundauflage unter Berücksichtigung der tatsächlich eingesetzten Werkstoffe und Dimensionen wieder. Die maximale Flüssigkeitsaüfnahmekapazität berechnet sich zu:

$$max.Fl. = (Flächengewicht \ x \ Flüssigkeitsaufnahme) + MVTR \left[\frac{g}{m^2 24h}\right] \quad (3)$$

6) Kontaktwinkelmessung T-Test 6
Kontaktwinkelmessungen wurden durch das Ostthüringische Materialprüfgesellschaft für Textil und Kunstoffe mbH, Rudolstadt - Deutschland, durchgeführt. Die Messung des Kontaktwinkels erfolgt mit Hilfe des DSA 100 Prüfgerätes der Firma Krüss, Hamburg - Deutschland, in Anlehnung an die DIN EN 828, wobei als Testflüssigkeit demineralisiertes Wasser dient. Zur Überprüfung der Kontaktwinkel wird das Elastomer auf einen handelsüblichen Polyurethan-Film (VP-940-2, Fa Collano, Buxtehude - Deutschland) vollflächig aufgetragen. Hierbei wird demineralisiertes Wasser auf dem Elastomer aufgetragen.

7) Trennkraftmessung Wundauflage von Modellwunde Glas/Fibrin - Test 7
Die Prüfung dient der Beurteilung wie gut sich eine Wundauflage von einer in-vitro Fibrinschicht lösen lässt. Folgende Reagenzien werden benötigt:

i) PBS-Puffer pH 7,4, Fa. Sigma-Aldrich, Steinheim - Deutschland, Art.Nr. P-5368. Der Inhalt eines Beutels wird mit demineralisiertem Wasser im Messkolben zu 1000 ml gelöst.
ii) Thrombin aus Rinderplasma, 50 NIH-U/mg, Fa. Merck, Darmstadt Deutschland, Art.-Nr. 112374. 10 mg Thrombin werden in ein Zentrifugenröhrchen aus PP eingewogen und mit 10 ml PBS-Pufferlösung, welche auf 37°C thermostatisiert wurde, auf dem Vortex-Schüttler gelöst. 1 Teil Trombin-Lösung wird mit 9 Teilen PBS-Puflerlösung verdünnt (= 5 NIH-U/ ml). Die Mischung muss für jeden Versuch neu angesetzt werden!
iii) 250 mg Fibrinogen (aus Humanplasma (95 % clottable protein), Fa. Sigma-Aldrich, Art.Nr. F-4883) werden in ein großes Zentrifugenröhrchen aus PP eingewogen und mit 100 ml PBS-Pufferlösung, welche auf 37°C thermostatisiert wurde, auf dem Vortex-Schüttler gelöst. Der tatsächliche Bedarf an Fibrinogenlösung muss entsprechend der

Zahl an Prüflingen angepasst werden. Die Lösung ist bei Raumtemperatur 48 h stabil. Die Glasplatte wird bei einer Länge von 16 cm markiert und an der Markierung mit einem Quersteg aus Schaumstoffdichtungsband (Tesa Moll Art.Nr. 05459-00047) versehen. Dann wird um den markierten Teil der Glasplatte eine Umrandung aus Schaumstoffdichtungsband gezogen und mit dem Quersteg flüssigkeitsdicht verklebt Die Wundauflage wird im Maß 160 x 50 mm ausgestanzt/abgeschnitten. 150 mg Fibrinogen werden in 60 ml PBS-Pufferlösung gelöst. Die Thrombinlösung wird hergestellt in dem 10 mg Thrombin in 10 ml PBS-Pufferlösung gelöst werden und von dieser Lösung 1 ml nochmals mit 9 ml PBS-Pufferlösung versetzt wird. Fibrinogen-Lösung und Thrombin-Lösung werden auf 37°C thermostatisiert. Für jede vorbereitete Glasplatte werden 25 ml Fibrinogenlösung und 2,5 ml Thrombinlösung benötigt. Dazu werden beide Lösungen gemischt und sofort ca. 10 s auf dem Vortex-Schüttler gemischt und dann auf die Glasplatte gegossen und gleichmäßig verteilt. Danach wird die so aufgebrachte Lösung 72 h bei Normklima (23°C, 50-% relative Feuchte) getrocknet. Vor der Trennkraftmessung wird die Schaumstoffumrandung von der Glasplatte entfernt.

Die Probe wird in der Dimension 100 x 40 mm ausgestanzt. Der Prüfkörper wird mit Hilfe eines Tape Applicators D 427/1 der Firma Sondes Place Research Institute, Surrey England mit 20 N pro cm definiert angerollt und 20 min Ruhen gelassen. Die so präparierte Glasplatte wird in die 90° Abzugsvorrichtung der Zug Dehnungsmaschine Z-005 der Firma Zwick-Roell, Ulm Deutschland, eingelegt und das freie Ende des Verstärkungsklebebandes in die obere Klemme eingespannt. Bei einer konstanten Abzugsgeschwindigkeit von 100 mm/min wird der Kraftverlauf gemessen. Nach dem Abziehen des Prüflings wird dieselbe Probe nach 20 Minuten nochmals wieder angerollt und wieder gemessen. Insgesamt sind 4 Messungen mit demselben Prüfkörper auf derselben Fibrinschicht vorzunehmen. Die Kraftauswertung erfolgt nach DIN ISO 6133, Verfahren C.

8) Prüfung Wundverklebung an der Modellwunde Agar/Fibrin (nach Deutsche Apotheker Zeitung 131. Jahrgang, Nr. 41, 2092-2094 (1991)) - Test 8

Im In-vitro-Versuch wird die Verklebungsneigung von Wundauflagen mit einem auf einer Agar-Oberfläche gebildeten Fibrinnetz geprüft. Folgende Reagenzien werden benötigt:

i) Agar-Agar, Art.Nr. 1.01614, Fa. Merck
ii) Humanplasma vom DRK-Blutspendedienst, portioniert und eingefroren bei-18°C
iii) Actin FS, Art.Nr. B4218-20 oder B4218-100, Fa. DADE BEHRING, Eschborn Deutschland.
iv) Calciumchlorid-Lösung 25 mM: 3,67 g Calciumchlorid-Dihydrat werden mit demineralisiertem Wasser zu 1000 ml gelöst.

Aus Agar-Agar und demineralisiertem Wasser wird durch Erhitzen bis zum Sieden eine 1,5 %ige Lösung hergestellt. Nach dem Abkühlen auf ca. 50°C werden jeweils 10 ml Lösung in eine Petrischale pipettiert und abgedeckt erkalten gelassen. Eine Portion Humanplasma wird im Wasserbad bei 37°C aufgetaut und temperiert.
Auf eine Agarplatte werden der Reihe nach pipettiert:

1. 750 $\mu$l Calciumchlorid-Lösung 25 mM
2. 1500 $\mu$l Humanplasma
3. 750 $\mu$l Actin FS

Die Substanzen werden durch Umschwenken gut gemischt und ohne Deckel für 30 Minuten im Klimaschrank bei 37°C / 50 % relative Feuchte thermostatisiert. In dieser Zeit beginnt das Fibrinnetz sich gelartig auf der Agarschicht zu bilden. Die zu prüfende Wundauflage wird auf eine Größe von 2 x 2 cm zugeschnitten. Die zugeschnittene Probe wird auf das flüssige und noch nicht vollständig gebildete Fibrinnetz gelegt und leicht angedrückt. Anschließend wird die Agarplatte mit der Wundauflage (ohne Deckel) für weitere 90 Minuten in den Klimaschrank gestellt.
Die Wundauflage wird nun mittels Pinzette von der Agarplatte entfernt und visuell oder mikroskopisch untersucht. Ausgewertet wird der Zustand des Fibrinnetzes nach Entfernen der Wundauflage. Hierbei wird eine Wundauflage als nicht wundverklebend bezeichnet, wenn

a) das Fibrinnetz nicht beschädigt wird oder
b) das Fibrinnetz wenige oder nur minimale Fehlstellen aufweist.

Dagegen wird eine Wundauflage als wundverklebend eingestuft, wenn

a) das Fibrinnetz beim Entfernen der Wundauflage reist oder beschädigt wird oder
b) Rückstände der Wundauflage im Fibrinnetz zurückgeblieben sind.

B) Erfindungsgemäße Zusammensetzung der Polyurethan-Elastomere

Tabelle 1 (Angaben in Gew.-%)

| Polyalkomponente (A) | Polyurethan-Elastomer 1: A | Polyurethan-Elastomer 2: A | Polyurethan-Elastomers 3: A | Polyurethan-Elastomer 4: A |
|---|---|---|---|---|
| Polyetherpolyol (Levagel VPKA 8732; Bayer); OHZ 35 | 99,4 | 99,4 | 99,4 | - |
| Trifunktionelles Polypropylenetherpolyol (Desmophen 5034 BT; Bayer) | - | - | - | 99,5 |
| DABCO 33LV (Air Products) | - | - | - | 0,5 |
| Bi(III)-Katalysator (hier: -neodecanoat; Coscat 83; Cosan Chemical Corporation) | 0.4 | 0.4 | 0,4 | - |
| Stabilisator (hier. Tocopherol) | 0.2 | 0.2 | 0,2 | - |
| Isocyanatkomponente (B) | Mischungsverhältnis A/B | Mischungsverhältnis A/B | Mischungsverhältnis A/B | Mischungsverhältnis A/B |
| Präpolymer auf Basis (HDI) (Desmodur E305; Bayer); NCO-Gehalt ca. 13 % | - | - | - | 2,48: 1,00 |
| Aliphatisches Präpolymer auf Basis (IPDI) (Desmodur VP LS 2371); NCO-Gehalt ca. 3,7 % | 2,70 : 1,00 | 3,45 : 1,00 | 2,34 : 1,00 | - |

Das Polyuerethan-Elastomer wird innerhalb kurzer Reaktionszeit erhalten, indem die beiden Komponenten bei Raumtemperatur zusammengegeben werden und homogen vermischt werden. Nach kurzer Zeit, in der die Reaktion zwischen den beiden Komponenten startet, erhöht sich zunehmend die Viskosität. Die Vervollständigung der Reaktion kann mittels zuführen von Wärme erfolgen.

C) Herstellung und Aufbau der Wundauflagen

Ausführungsbeispiel 1:

Als Wundkontaktschicht wurde das klebende Polyurethan-Elastomer 1 (Firma Collano, Sempach-Station - Schweiz) benutzt. Dieser Haftklebstoff besteht aus einer Polyether-Polyol Komponente (A) und einer cycloaliphatischen Isocyanatkomponente (B). Es wurden 100 g der Komponente A mit 37 g der Komponente B homogen gemischt. Diese Mischung wurde auf einem Silikonpapier Separacon 9120-60 der Firma Maria Soell, Nidderau - Deutschland, mit einer Schichtdicke von etwa 200 $\mu$m vollflächig aufgetragen. Nach kurzer Zeit, in der die Reaktion zwischen den beiden Komponenten startet, erhöht sich zunehmend die Viskosität. Daraufhin wurde zu dieser sich formenden Polyurethanschicht PermaFoam (Dicke ca. 5 mm, Art.-Nr. 409401) der Firma Paul Hartmann AG, Heidenheim - Deutschland zulaminiert. Im Folgenden wurde eine Wundauflage der Größe 10 x 10 cm ausgestanzt. Damit entspricht der Aufbau dieser Wundauflage dem mit Figur 1 schematisch dargestellten Aufbau.

Tabelle 21: Eigenschaften des Ausführungsbeispiels 1:

| Testmethode | Einheit | Beispiel 1 | PermaFoam |
|---|---|---|---|
| Freie Absorption (Test 3)) | g/g | 9,6 | 10,1 |
| Wasserdampfdurchlässigkeit (Test 4a) | g/ m$^2$/24 h | 307 | 301 |
| Flüssigkeitsaufnahmekapazität (Test 5) | g/ m$^2$/24 h | 6451 | 6765 |
| Klebkraft 90° (Test 1) | N/25 mm | 0,09 | - |
| Kontaktwinkel (Test 6) | ° | - [1] | - |
| [1] - nicht messbar, da plötzliches Aufspreiten des Tropfens nach wenigen Sekunden beobachtet wird | | | |

Ausführungsbeispiel 1 zeigt, das bei zusätzlichem Aufbringen von einer hydrophilen Wundkontaktschicht, die Leistungseigenschaften einer Wundauflage ohne Wundkontaktschicht nicht verändert oder nicht wesentlich verändert werden. Hierbei sind insbesondere die Wasserdampfdurchlässigkeit und die maximale Flüssigkeitsaufnahmekapazität hervorzuheben (vgl. Tabelle 2). Die hydrophile Wundkontaktschicht des Beispiels 1 besitzt eine geringe Klebkraft auf Stahl, wobei sich in einem Tragetest herausgestellt hat, dass ein Haftvermögen auf trockener Haut für eine Erstfixierung der Wundauflage ausreichend ist. Das Ausführungsbeispiel 1 zeigte gemäß der Testmethode "Prüfung Wundverklebung an der Modellwunde Agar/Fibrin" (Test 8) zudem keine Beschädigung der Fibrinschicht nach Abzug der Wundauflage. Die Wundauflage ist somit als nicht wundverklebend einzustufen.

Tabelle 3: Prüfung auf mehrmaliges Aufkleben des Ausführungsbeispiels 1 auf dieselbe Oberfläche gemäß Testmethode Test 7:

| Testlauf /min | Mittlere Kraft /N/40 mm | Maximal gemessens Kraft /N/40 mm |
|---|---|---|
| 1. Abzug t=0 | 0,39 | 0,56 |
| 2. Abzug t+20 min | 0,37 | 0,53 |
| 3. Abzug t+40 min | 0,38 | 0,53 |
| 4. Abzug t+60 min | 0,39 | 0,56 |

Das Ausführungsbeispiel 1 zeigte gemäß der Testmethode Test 7, dass diese Wundauflage auch nach wiederholtem Abnehmen der Wundauflage seine Klebkraft nicht verliert.

Ausführungsbeispiel 2 (zur Bestimmung einzelner Eigenschaften der Wundkontaktschicht oder des hydrophilen Polyurethan-Elastomers)

Polyurethan-Elastomer 1 wurde auf einem Polyurethan-Film (VP940-2) der Firma Collano-Xiro, Buxtehude - Deutschland mit einem Auftragsgewicht von 200 g/ m$^2$ beschichtet. Dieses Laminat wurde hergestellt, um die freie Absorption des Polyurethan-Elastomer sowie dessen Kontaktwinkel zu bestimmen. Zur Bestimmung der freien Absorption wurde das Gewicht des Polyurethan-Films abgezogen. Die Bestimmung der freien Absorption erfolgte gemäß Test 3. Die Testungen werden frühestens 3 Tage nach Fertigstellung des Laminats durchgeführt.

Ausführungsbeispiel 3 (zur Bestimmung einzelner Eigenschaften der Wundkontaktschicht oder des hydrophilen Polyurethan-Elastomers)

Polyurethan-Elastomer 2 wurde auf einem PU-Trägerfilm (Polyurethan-Film VP940-2, Firma Collano-Xiro, Buxtehude - Deutschland) mit einem Auftragsgewicht von 60 g/ m$^2$ beschichtet. Dieses Laminat wurde hergestellt, um die freie Absorption des Polyurethan-Elastomer sowie dessen Kontaktwinkel zu bestimmen. Zur Bestimmung der freien Absorption wurde das Gewicht des Polyurethan-Films abgezogen. Die Bestimmung der freien Absorption erfolgt gemäß Test 3. Die Testungen werden frühestens 3 Tage nach Fertigstellung des Laminats durchgeführt.

Tabelle 4: Eigenschaften der Ausführungsbeispielen 2 und 3

| | Absorption /g/g (Test 3) | Kontaktwinkel /° (Test 6) | MVTR /g/m$^2$/24h (Test 4a) | MVTR[#] /g/m$^2$/24h (Test 4b) | Klebkraft /N/ 25 mm (Test 2) |
|---|---|---|---|---|---|
| Beispiel 2 | 0,92 | - [1] | 1331 | 2361 | 0,20 |
| Beispiel 3 | 0,60 | 63 [2] | 1487 | 2745 | 2,84 |
| PU-Trägerfilm | 0 | - | 2598 | 4317 | - |

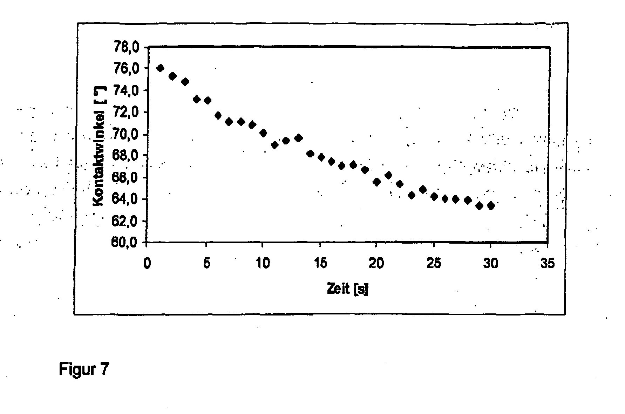

[#] Bei einem Auftragsgewicht von 100 g/ m2
[1] - nicht messbar, da plötzliches Aufspreiten des Tropfens nach wenigen Sekunden beobachtet wird; die Oberfläche ist damit als sehr hydrophil zu bezeichnen
[2] - Kontaktwinkel nach 30 Sekunden nach Aufbringung des Tropfens auf die klebende PU-Gel-Seite des Ausführungsbeispieles 3. Die starke Veränderung des Kontaktwinkel in Abhängigkeit der Zeit zeigt den hydrophilen Charakter von Ausführungsbeispiel 3 (vergleiche Figur 7)

Mit diesen Beispielen kann gezeigt werden, dass die verwendeten Elastomere bzw. die Wundkontaktschichten hydrophil sind und eine gute freie Absorption sowie eine gute Wasserdampfdurchlässigkeit besitzen.

**Patentansprüche**

1. Mehrschichtige Wundauflage (10, 20, 30, 40, 50, 60) umfassend eine Trägerschicht (13, 23, 33, 43, 53, 63), eine absorbierende Schicht (12, 22, 32, 42, 52, 62) und eine hydrophile Wundkontaktschicht (11, 21, 31, 41, 51, 61), wobei die Wundkontaktschicht mit der absorbierenden Schicht verbunden ist und ein hydrophiles Polyurethan-Elastomer umfasst, **dadurch gekennzeichnet, dass** das Elastomer ein klebendes Polyurethan-Elastomer oder ein Polyurethan-Elastomer-Haftklebstoff ist, welches erhältlich ist durch eine Polymerisation von wenigstens einer aliphatischen und/ oder cycloaliphatischen Isocyanat-Komponente mit einer Polyetherpolyol-Komponente.

2. Wundauflage (10, 30) nach Anspruch 1, **dadurch gekennzeichnet, dass** die absorbierende Schicht (12, 32) integral mit der Wundkontaktschicht (11, 31) verbunden ist.

3. Wundauflage (10, 20, 30, 40, 50, 60) nach mindestens einem der vorangehenden Ansprüche, **dadurch gekennzeichnet, dass** die Wundauflage als Wundkontaktschicht ein Polyurethan-Elastomer enthält, insbesondere ein Polyurethan-Elastomer-Haftklebstoff, welches erhältlich ist durch die Polymerisation von Isophorondiisocyanat oder einem modifizierten Isophorondiisocyanat mit wenigstens einer Diol- oder Pololkomponente, vorzugsweise 2, 3, 4, 5 oder 6 Hydroxylgruppen aufweisenden Polyetherpolyolen mit OH-Zahlen von 20 bis 112 und einem Ethylenoxid (EO)-Gehalt von ≥ 10 Gew.%, vorzugsweise 10 bis 40 Gew.%, besonders bevorzugt 10 bis 20 Gew.%.

**4.** Wundauflage (20, 40, 60) nach mindestens einem der vorangehenden Ansprüche, **dadurch gekennzeichnet, dass** die Wundkontaktschicht (21, 41, 61) eine Klebkraft von 0,02 bis 5 N/ 25 mm aufweist.

**5.** Wundauflage (50) nach mindestens einem der vorangehenden Ansprüche, **dadurch gekennzeichnet, dass** die Wundkontaktschicht (51) eine diskontinuierliche Wundkontaktschicht ist.

**6.** Wundauflage (10, 20, 30, 40, 50, 60) nach mindestens einem der vorangehenden Ansprüche, **dadurch gekennzeichnet, dass** die Wundauflage als absorbierende Schicht einen hydrophilen Polymer-Schaum, ein absorbierendes Vlies oder Nonwoven, eine mindestens ein Hydrocolloid umfassende Polymermatrix, einen gefriergetrockneten Schaum oder Kombinationen hiervon umfasst.

**7.** Wundauflage (10, 20, 30, 40, 50, 60) nach mindestens einem der vorangehenden Ansprüche, **dadurch gekennzeichnet, dass** die absorbierende Schicht einen hydrophilen Polyurethanschaum umfasst.

**8.** Wundauflage (10, 20, 30, 40, 50, 60) nach mindestens einem der vorangehenden Ansprüche, **dadurch gekennzeichnet, dass** die Wundauflage eine maximale Flüssigkeitsaufnahmekapazität von mindestens 5000 g/ m$^2$/ 24 h aufweist.

**9.** Wundauflage (10, 20, 30, 40, 50, 60) nach mindestens einem der vorangehenden Ansprüche, **dadurch gekennzeichnet, dass** die Trägerschicht (13, 23, 33, 43, 53, 63) aus einem Polyurethanschaum oder Polyurethanfilm besteht.

**10.** Wundauflage (10, 20, 30, 40, 50, 60) nach mindestens einem der vorangehenden Ansprüche, **dadurch gekennzeichnet, dass** die Trägerschicht (13, 23, 33, 43, 53, 63) und die absorbierende Schicht zusammen aus einem Laminat aus zwei verschiedenen Polyurethanschäumen besteht.

**11.** Wundauflage (10, 20, 30, 40, 50, 60) nach mindestens einem der vorangehenden Ansprüche, **dadurch gekennzeichnet, dass** die absorbierende Schicht eine höhere freie Absorption aufweist als die Wundkontaktschicht.

**12.** Mehrschichtige Wundauflage (60) nach mindestens einem der vorangehenden Ansprüche, **dadurch gekennzeichnet, dass** die hydrophile Wundkontaktschicht (61) weiterhin mindestens ein antimikrobielles Mittel (68) umfasst.

**13.** Mehrschichtige Wundauflage (30) nach mindestens einem der vorangehenden Ansprüche, **dadurch gekennzeichnet, dass** die Wundauflage weiterhin mindestens eine Verteilerschicht (36) umfasst.

**Claims**

**1.** Multi-layer wound dressing (10, 20, 30, 40, 50, 60) comprising a carrier layer (13, 23, 33, 43, 53, 63), an absorbent layer (12, 22, 32, 42, 52, 62) and a hydrophilic wound contact layer (11, 21, 31, 41, 51, 61), wherein the wound contact layer is connected to the absorbent layer and comprises a hydrophilic polyurethane elastomer, **characterized in that** the elastomer is an adhesive polyurethane elastomer or a polyurethane elastomer pressure-sensitive adhesive and is obtainable by a polymerization of at least one aliphatic and/or cycloaliphatic isocyanate component with a polyether polyol component.

**2.** Wound dressing (10, 30) according to Claim 1, **characterized in that** the absorbent layer (12, 32) is integrally connected to the wound contact layer (11, 31).

**3.** Wound dressing (10, 20, 30, 40, 50, 60) according to at least one of the preceding claims, **characterized in that** the wound contact layer comprises a polyurethane elastomer, particularly a polyurethane elastomer pressure-sensitive adhesive, wherein the elastomer is obtainable by the polymerization of isophorone diisocyanate or of a modified isophorone diisocyanate with at least one diol or polyol component, preferably polyether polyols containing 2, 3, 4, 5 or 6 hydroxyl groups and having OH numbers of 20 to 112 and an ethylene oxide (EO) content of $\geq$ 10% by weight, preferably 10% to 40% by weight and more preferably 10% to 20% by weight.

**4.** Wound dressing (20, 40, 60) according to at least one of the preceding claims, **characterized in that** the wound contact layer (21, 41, 61) has an adhering force of 0.02 to 5 N/25 mm.

**5.** Wound dressing (50) according to at least one of the preceding claims, **characterized in that** the wound contact layer (51) is a discontinuous wound contact layer.

**6.** Wound dressing (10, 20, 30, 40, 50, 60) according to at least one of the preceding claims, **characterized in that** the absorbent layer comprises a hydrophilic polymer foam, an absorbent fibrous web or nonwoven, a polymer matrix comprising at least one hydrocolloid, a freeze-dried foam or combinations thereof.

**7.** Wound dressing (10, 20, 30, 40, 50, 60) according to at least one of the preceding claims, **characterized in that** the absorbent layer comprises a hydrophilic polyurethane foam.

**8.** Wound dressing (10, 20, 30, 40, 50, 60) according to at least one of the preceding claims, **characterized in that** the wound dressing has a maximum liquid handling capacity of at least 5000 g/m$^2$/24 h.

**9.** Wound dressing (10, 20, 30, 40, 50, 60) according to at least one of the preceding claims, **characterized in that** the carrier layer (13, 23, 33, 43, 53, 63) consists of a polyurethane foam or polyurethane film.

**10.** Wound dressing (10, 20, 30, 40, 50, 60) according to at least one of the preceding claims, **characterized in that** the carrier layer (13, 23, 33, 43, 53, 63) and the absorbent layer together consists of a laminate of two different polyurethane foams.

**11.** Wound dressing (10, 20, 30, 40, 50, 60) according to at least one of the preceding claims, **characterized in that** the absorbent layer has a higher free absorption than the wound contact layer.

**12.** Multi-layer wound dressing (60) according to at least one of the preceding claims, **characterized in that** the hydrophilic wound contact layer (61) further comprises at least one antimicrobial agent (68).

**13.** Multi-layer wound dressing (30) according to at least one of the preceding claims, **characterized in that** the wound dressing further comprises at least one distributor layer (36).

**Revendications**

**1.** Pansement multicouche (10, 20, 30, 40, 50, 60), comprenant une couche support (13, 23, 33, 43, 53, 63), une couche absorbante (12, 22, 32, 42, 52, 62) et une couche hydrophile de contact avec la plaie (11, 21, 31, 41, 51, 61), la couche de contact avec la plaie étant reliée avec la couche absorbante et comprenant un élastomère de polyuréthane hydrophile, **caractérisé en ce que** l'élastomère est un élastomère de polyuréthane adhésif ou un adhésif sensible à la pression à base d'un élastomère de polyuréthane, qui peut être obtenu par une polymérisation d'au moins un composant isocyanate aliphatique et/ou cycloaliphatique avec un composant polyéther-polyol.

**2.** Pansement (10, 30) selon la revendication 1, **caractérisé en ce que** la couche absorbante (12, 32) est reliée intégralement avec la couche de contact avec la plaie (11, 31).

**3.** Pansement (10, 20, 30, 40, 50, 60) selon au moins l'une quelconque des revendications précédentes, **caractérisé en ce que** le pansement contient en tant que couche de contact avec la plaie un élastomère de polyuréthane, notamment un adhésif sensible à la pression à base d'un élastomère de polyuréthane, qui peut être obtenu par la polymérisation de diisocyanate d'isophorone ou d'un diisocyanate d'isophorone modifié avec au moins composant diol ou polyol, de préférence des polyéther-polyols comportant 2, 3, 4, 5 ou 6 groupes hydroxyle, ayant des indices OH de 20 à 112 et une teneur en oxyde d'éthylène (OE) ≥ 10 % en poids, de préférence de 10 à 40 % en poids, de manière particulièrement préférée de 10 à 20 % en poids.

**4.** Pansement (20, 40, 60) selon au moins l'une quelconque des revendications précédentes, **caractérisé en ce que** la couche de contact avec la plaie (21, 41, 61) présente une force adhésive de 0,02 à 5 N/25 mm.

**5.** Pansement (50) selon au moins l'une quelconque des revendications précédentes, **caractérisé en ce que** la couche de contact avec la plaie (51) est une couche de contact avec la plaie discontinue.

**6.** Pansement (10, 20, 30, 40, 50, 60) selon au moins l'une quelconque des revendications précédentes, **caractérisé en ce que** le pansement contient en tant que couche absorbante une mousse de polymère hydrophile, un mat ou non-tissé absorbant, une matrice polymère comprenant au moins un hydrocolloïde, une mousse lyophilisée ou leurs combinaisons.

7. Pansement (10, 20, 30, 40, 50, 60) selon au moins l'une quelconque des revendications précédentes, **caractérisé en ce que** la couche absorbante comprend une mousse de polyuréthane hydrophile.

8. Pansement (10, 20, 30, 40, 50, 60) selon au moins l'une quelconque des revendications précédentes, **caractérisé en ce que** le pansement présente une capacité maximale d'absorption des liquides d'au moins 5 000 g/m$^2$/24 h.

9. Pansement (10, 20, 30, 40, 50, 60) selon au moins l'une quelconque des revendications précédentes, **caractérisé en ce que** la couche support (13, 23, 33, 43, 53, 63) est constituée d'une mousse de polyuréthane ou d'un film de polyuréthane.

10. Pansement (10, 20, 30, 40, 50, 60) selon au moins l'une quelconque des revendications précédentes, **caractérisé en ce que** la couche support (13, 23, 33, 43, 53, 63) et la couche absorbante sont constituées ensemble d'un stratifié de deux mousses de polyuréthane différentes.

11. Pansement (10, 20, 30, 40, 50, 60) selon au moins l'une quelconque des revendications précédentes, **caractérisé en ce que** la couche absorbante présente une absorption libre supérieure à la couche de contact avec la plaie.

12. Pansement multicouche (60) selon au moins l'une quelconque des revendications précédentes, **caractérisé en ce que** la couche hydrophile de contact avec la plaie (61) comprend également au moins un agent antimicrobien (68).

13. Pansement multicouche (30) selon au moins l'une quelconque des revendications précédentes, **caractérisé en ce que** le pansement comprend également au moins une couche de distribution (36).

Figur 1

Figur 2

Figur 3

Figur 4

Figur 5

Figur 6

Figur 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 59048 A
- EP 59049 A
- EP 97517 A
- EP 99748 A
- EP 106439 A
- EP 486522 A
- WO 9407935 A
- WO 9742985 A
- WO 2004060359 A
- EP 1007597 B1